(19) Europäisches Patentamt
European Patent Office
Office européen des brevets

(11) EP 2 649 921 A1

(12) EUROPEAN PATENT APPLICATION
published in accordance with Art. 153(4) EPC

(43) Date of publication:
16.10.2013 Bulletin 2013/42

(51) Int Cl.:
A61B 1/00 (2006.01)     G02B 23/24 (2006.01)

(21) Application number: 12751928.8

(22) Date of filing: 13.02.2012

(86) International application number:
PCT/JP2012/053244

(87) International publication number:
WO 2012/117836 (07.09.2012 Gazette 2012/36)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 28.02.2011 JP 2011042552

(71) Applicant: Olympus Medical Systems Corp.
Tokyo 151-0072 (JP)

(72) Inventor: OKAMOTO, Yasuhiro
Tokyo 151-0072 (JP)

(74) Representative: Gunzelmann, Rainer
Wuesthoff & Wuesthoff
Patent- und Rechtsanwälte
Schweigerstraße 2
81541 München (DE)

(54) **MEDICAL DEVICE EQUIPPED WITH BOWING-SECTION, AND ENDOSCOPE**

(57) A medical apparatus equipped with a bending portion is a medical apparatus endoscope equipped with a bending portion, comprising: a running path changing member adapted to change running paths of a plurality of pulling members extended from bending pieces constituting a bending portion and led into an operation section; a suspension frame made up of a plurality of frames and configured such that a proximal end portion of a pulling member whose running path is changed by the running path changing member is fixedly mounted on a pulling member mounting portion provided at an end portion of each frame; and an operation lever integrally fixed to the suspension frame and provided with an axial portion which is installed by protruding orthogonally to a longitudinal axis of an operation section body constituting the operation section and is adapted to undergo a tilting operation, wherein an angle at which the pulling member fixedly mounted on the pulling member mounting portion enters the running path changing member is set for each of the pulling members.

**FIG.6**

EP 2 649 921 A1

**Description**

Technical Field

The present invention relates to a medical apparatus equipped with a bending portion and an endoscope which are provided with a bendable bending portion on a distal end side of an insertion portion.

Background Art

Recently, endoscopes equipped with an elongated insertion portion have been used in a medical field or an industrial field. The endoscope in the medical field allows observation by inserting the insertion portion into the body through the oral cavity, anus, or the like. On the other hand, the endoscope in the industrial field allows observation by inserting the insertion portion into boiler piping, an inner part of an engine, or the like.

In the endoscope, a bending portion adapted to bend, for example, in up, down, left, and right directions is provided on a distal end side of the insertion portion to allow an observation optical system provided in the distal end portion of the insertion portion to be oriented in a desired direction. A bending knob for bending operation of the bending portion is pivotally disposed in an operation section provided at a proximal end of the insertion portion. An angle wire is coupled to a predetermined location of the bending portion and to a predetermined location of the bending operation knob. The endoscope configured in this way is designed to allow the bending portion to be bent as an operator pulls or slackens the angle wire by manually rotating the bending operation knob clockwise or counterclockwise.

For example, in an endoscope shown in Fig. 1(A) of Japanese Patent Application Laid-Open Publication No. 2000-051146 (hereinafter referred to as Document 1), a UD bending operation knob used to operate and bend the bending portion in up and down directions and an RL bending operation knob used to operate and bend the bending portion in left and right directions are disposed coaxially in an operation section casing. The endoscope is configured to allow the operator to bend the bending portion in an up and down direction by rotating the UD bending operation knob and bend the bending portion in a left and right direction by rotating the RL bending operation knob. While gripping the operation section casing with fingers of a hand, the operator can bend the bending portion by operating and rotating the bending operation knob with the gripping fingers.

However, it is laborious to perform an operation for directing the distal end portion of the endoscope to a desired direction while operating the two bending operation knobs by the fingers which grip the operation section casing. Thus, there has been a problem that the operation of the two bending operation knobs is difficult for a doctor having a small hand such as a female doctor, etc.

Also, in order to overcome this problem, various types of endoscopes have been proposed which incorporate an electric motor in the operation section of the endoscope to allow bending motion of the bending portion to be performed by operating an operation lever, which is a bending mechanism, with a single finger.

For example, an electrically-bent endoscope is shown in Japanese Patent Application Laid-Open Publication No. 2004-321612 (hereinafter referred to as Document 2). With the electrically-bent endoscope, when a joystick of a joystick device, which is an operation lever provided in the operation section, is operated and tilted from a neutral position, a bending operation wire is pulled or slackened under a driving force of a motor provided in the operation section, thereby bending the bending portion by an amount corresponding to the tilting operation.

Also, Japanese Patent Application Laid- Open Publication No. 2005- 279118 (hereinafter referred to as Document 3) discloses an active tube drive apparatus embodied in an active tube drive system. With the active tube drive system, when a distal end portion of a control information input section, which is an operation lever of a controller, is operated by hand, a value of a variable resistor provided in the control information input section is inputted and a control element in the controller is controlled according to the input. The control element uses PWM control to change an amount of current conducted to an SMA coil in a distal end portion of a catheter. Consequently, a flexing mechanism acts in response to the amount of current conducted to the SMA coil and thereby keeps a flexion angle of the distal end portion constant.

Also, an endoscope equipped with a pulling member operation apparatus is shown in Japanese Patent Application Laid- Open Publication No. 2003- 325437 (hereinafter referred to as Document 4) . The endoscope allows a bending portion to be operated and bent by operating and tilting an operator control lever, which is an operation lever, with a slight amount of operating force and thereby moving a desired pulling member by a desired amount. The endoscope allows a bending portion to be bent through a tilting operation of a bending lever: the tilting operation changes tensioned states of operation wires fixed to an arm member and adapted to respond to the tilting operation, thereby changes drag of an appropriate operation wire on a pulley rotated by a motor, and thereby moves the operation wire to a rotation direction of the pulley, bending the bending portion.

However, in Document 1, it is designed such that when the operator operates the UD bending operation knob or RL bending operation knob, the bending portion performs a bending motion in a rotation direction of the selected bending operation knob, whereas in Documents 2-4, it is designed such that when the operator performs a tilting operation, the bending portion performs a bending motion in a direction corresponding to a tilting direction. Therefore, when the operation for bending the bending portion is performed in a state where a posture of the operator or a gripping state of the operation section has been changed during an endoscopic observation, in Document 1, the bending portion is bent according to a rotational direction of the bending operation knob selected by the operator. That is, the bending portion is bent in the direction intended by the operator irrespective of the posture of the operator. On the other hand, in Documents 2-4, when the operator operates the operation lever to be tilted, there is a fear that the operation lever is displaced with respect to the tilting direction intended by the operator depending on the posture of the operator.

The present invention has been made in view of the above circumstances, and an object of the present invention is to provide a medical apparatus equipped with a bending portion and an endoscope which are capable of bending a bending portion by causing an operation lever to tilt in a direction intended by an operator by sensuously grasping a tilting direction of the operation lever when the operator causes the bending portion to bend by operating the operation lever to tilt.

Disclosure of Invention

Means for Solving the Problem

A medical apparatus equipped with a bending portion according to one aspect of the present invention comprises: a running path changing member adapted to change running paths of a plurality of pulling members extended from bending pieces constituting the bending portion and led into an operation section; a suspension frame made up of a plurality of frames and configured such that a proximal end portion of a pulling member whose running path is changed by the running path changing member is fixedly mounted on a pulling member mounting portion provided at an end portion of each frame; and an operation lever integrally fixed to the suspension frame and provided with an axial portion which is installed by protruding orthogonally to a longitudinal axis of an operation section body constituting the operation section and is adapted to undergo a tilting operation, wherein an angle at which the pulling member fixedly mounted on the pulling member mounting portion enters the running path changing member is set for each of the pulling members.

An endoscope according to one aspect of the present invention is a medical apparatus endoscope equipped with a bending portion, comprising: a running path changing member adapted to change running paths of a plurality of pulling members extended from bending pieces constituting a bending portion and led into an operation section; a suspension frame made up of a plurality of frames and configured such that a proximal end portion of a pulling member whose running path is changed by the running path changing member is fixedly mounted on a pulling member mounting portion provided at an end portion of each frame; and an operation lever integrally fixed to the suspension frame and provided with an axial portion which is installed by protruding orthogonally to a longitudinal axis of an operation section body constituting the operation section and is adapted to undergo a tilting operation, wherein an angle at which the pulling member fixedly mounted on the pulling member mounting portion enters the running path changing member is set for each of the pulling members.

Brief Description of the Drawings

Figs. 1 to 8 concern an embodiment of the present invention, where Fig. 1 is a diagram illustrating an endoscope which is an example of a medical apparatus equipped with a bending portion, in which an operation lever of a pulling member operation apparatus is installed upright on an operation section;

Fig. 2 is a diagram illustrating a configuration of a pulling member operation apparatus in which an operation section is made up of a grasping portion and an operation section body, with a motor and a pulley incorporated in the grasping portion;

Fig. 3 is a diagram illustrating a rotating body;

Fig. 4 is a diagram illustrating a configuration of the pulling member operation apparatus as viewed in a direction of an arrow Y4 in Fig. 2;

Fig. 5 is a diagram illustrating angles at which individual bending wires connected to a suspension frame enter respective guide rollers;

Figs. 6(a) and 6(b) are diagrams illustrating a relationship between multiple rotating bodies placed on the pulley and the suspension frame as viewed in a direction of an arrow Y6a in Fig. 5 and in a direction of an arrow Y6-Y6 in Fig. 6(a);

Fig. 7 is a diagram illustrating a relationship between an amount of upward tilting operation force and an amount of upward wire pulling force as well as a

relationship between an amount of downward tilting operation force and an amount of downward wire pulling force; and

Fig. 8 is a diagram illustrating an adjustment example of adjusting a length of an upper frame based on the relationship between the amount of upward tilting operation force and the amount of upward wire pulling force as well as the relationship between the amount of downward tilting operation force and the amount of downward wire pulling force.

Best Mode for Carrying Out the Invention

An embodiment of the present invention will be described below with reference to the drawings.

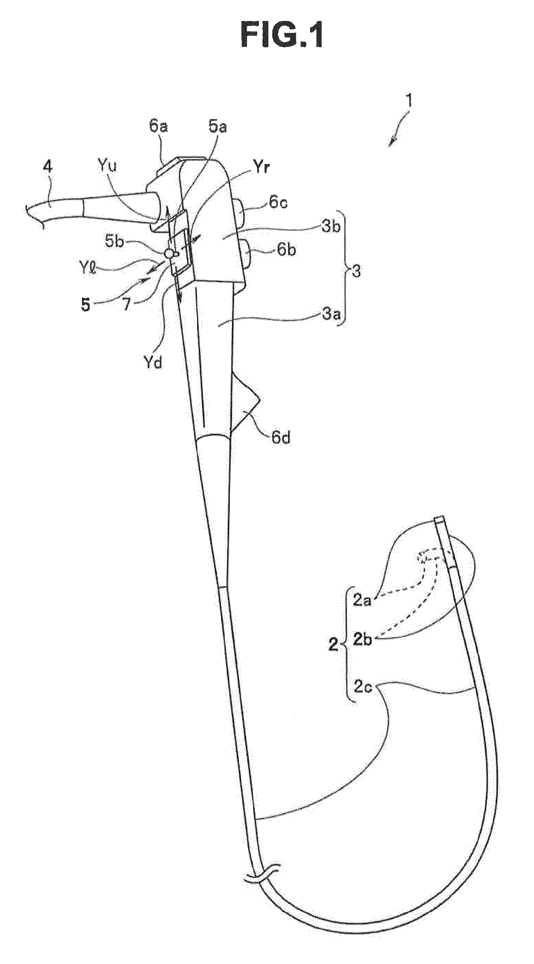

The embodiment of the present invention will be described with reference to Figs. 1 to 8.

According to the present embodiment, a medical apparatus equipped with a bending portion is an endoscope. As shown in Figs. 1 to 6, the endoscope 1 according to the present embodiment includes an elongated insertion portion 2, an operation section 3 installed consecutively with a proximal end of the insertion portion 2, and a universal cord 4 which extends form a flank of the operation section 3.

Starting from a distal end side, the insertion portion 2 includes a distal end portion 2a, a bending portion 2b, and a flexible tubular portion 2c, all of which are installed consecutively. The distal end portion 2a incorporates an image pickup apparatus (not shown) having an image pickup device. The bending portion 2b is configured to be bendable, for example, in up, down, left, and right directions. The flexible tubular portion 2c has flexibility and a long length.

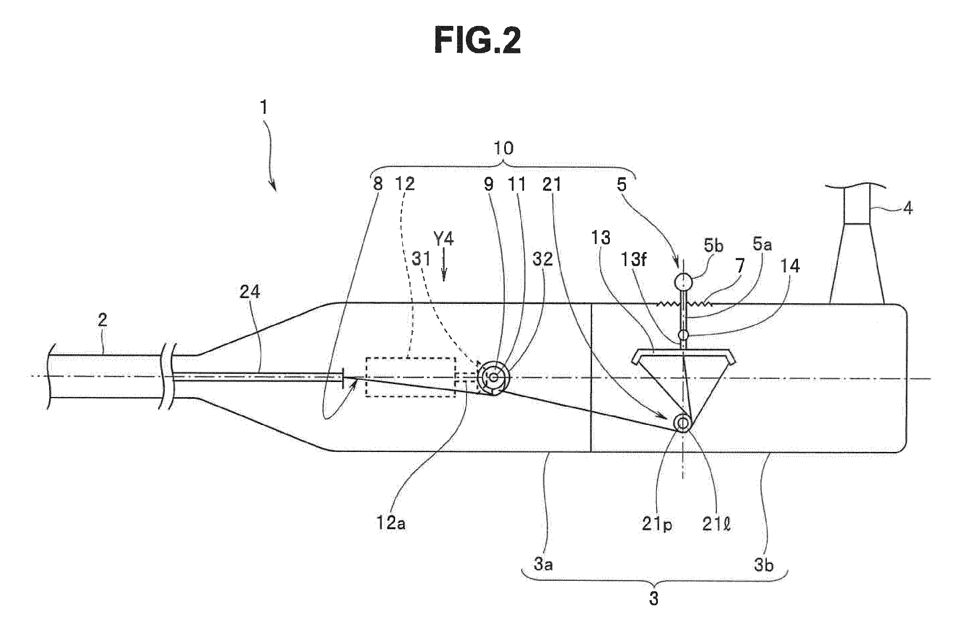

As shown in Figs. 1 and 2, the operation section 3 includes a grasping portion 3a and an operation section body 3b. The grasping portion 3a is installed consecutively with the insertion portion 2 while the operation section body 3b is installed consecutively with the grasping portion 3a. A longitudinal axis of the grasping portion 3 a and an insertion axis of the insertion portion 2 are in such a  positional relationship as to be coaxial or parallel with each other. An operation lever 5 for performing an operation to cause the bending portion 2b to perform bending motion is provided at a location corresponding to that part on a distal end side of the operation section body 3b in which the largest free space is available. A longitudinal axis of the operation section body 3b (also referred to as a longitudinal axis of the operation section 3) and a longitudinal axis of the grasping portion 3a are in such a positional relationship as to be coaxial or parallel with each other.

The operation lever 5 is installed orthogonally to the longitudinal axis of the operation section 3 through an operation lever projection hole (not shown) which is an opening provided in one face of the operation section body 3b.

The bending portion 2b is configured to bend according to tilting operation including a tilting direction and a tilting angle of the operation lever 5 as indicated by arrows Yu, Yd, Y1, and Yr in Fig. 1. Specifically, the bending portion 2b bends in an up direction, a right direction, a down direction, a left direction, a direction between the up direction and the right direction, and the like when bending operation wires (hereinafter abbreviated to bending wires) described later are pulled or slackened through tilting operation of the operation lever 5.

According to the present embodiment, the bending portion 2b is configured to bend in four directions: up, down, left, and right directions. However, the bending portion 2b may be configured to bend in up and down directions. The characters u, d, 1, and r above represent the up, down, left, and right directions, which are bending directions of the bending portion 2b. In the following description, for example, reference character 8u denotes an upward-acting bending wire and reference character 9d denotes a rotating body for a downward-acting bending wire. Besides, in drawings, a lower-case "1" is written in cursive to distinguish the letter from the numeral "1."

Incidentally, as shown in Fig. 1, a switch 6a, an air/ water feed button 6b, and a suction button 6c are provided at preset locations on an exterior of the operation section body 3b in addition to the operation lever 5. The switch 6a is used, for  example, to specify various image pickup actions of the image pickup apparatus provided in the distal end portion 2a. Also, a channel entrance port 6d communicated with a treatment instrument channel (not shown) is provided in an exterior of the grasping portion 3a.

According to the present embodiment, when the operator grips the grasping portion 3 a of the operation section 3 with the left hand as in the case of a conventional endoscope, the operation lever 5 is provided at such a location as to be operated with the thumb of the operator's griping hand, the air/water feed button 6b and the suction button 6c are provided at such locations as to be operated with fingers of the operator's griping hand other than the thumb, and the switch 6a is provided at such a location as to be operated with the thumb or other fingers of the operator's griping hand.

In Figs. 1 and 2, reference numeral 7 denotes a cover member. The cover member 7 closes the operation lever projection hole in a watertight fashion and tiltably holds the operation lever 5 in close contact with an axial portion 5a.

A signal cable, electric wires, a light guide fiber bundle, an air feed tube, a water feed tube, and a suction tube are passed through the universal cord 4. The signal cable is connected to the image pickup apparatus. The electric wires supply electric power to a motor (see reference numeral 12 in Fig. 2) described later. The light guide fiber bundle transmits illuminating light from a light source device.

As shown in Fig. 2, a pulling member operation apparatus 10 is provided in the operation section 3. The pulling member operation apparatus 10 mainly includes four bending wires 8, an elongated pulley 11 with four rotating bodies 9 mounted thereon, a motor 12 which is drive means, a substantially cross-shaped suspension frame 13, the operation lever 5, and a guide roller set 21 made up of multiple guide rollers described later.

The bending wires 8 are pulling members. Mid portions of the individual wires 8 are wound around the respective rotating bodies 9. The motor 12 develops a driving force for rotating a predetermined one of the rotating bodies 9 disposed on the pulley 11 during bending operation, with predetermined torque. The suspension frame 13 includes wire mounting portions to which proximal end portions of the respective wires 8 are coupled. The axial portion 5a of the operation lever 5 is integrally coupled to the suspension frame 13. The multiple guide rollers of the guide roller set 21 are wire running path changing members used to change running paths of the four wires 8 in the operation section 3.

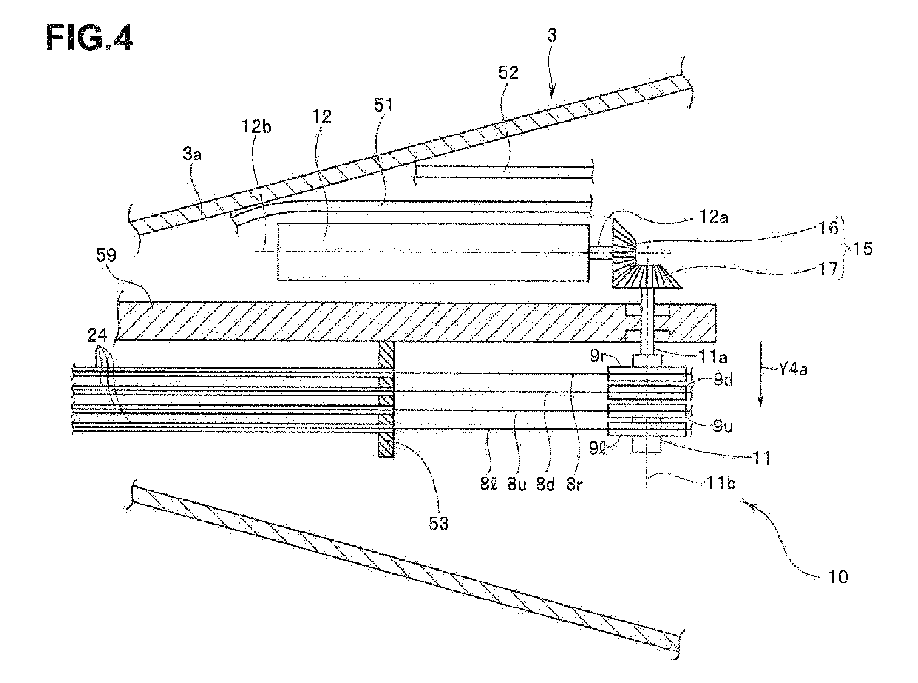

Incidentally, in Fig. 4, reference numeral 51 denotes a signal cable, reference numeral 52 denotes a light guide cable, reference numeral 53 denotes a coil pipe clamp, and reference numeral 59 denotes a partition plate. The present embodiment is configured such that the center of gravity of the operation section 3 will be located in the grasping portion 3a.

The four bending wires 8 are a pair of an upward-acting bending wire (hereinafter referred to as an Up bending wire) 8u and a downward-acting bending wire (hereinafter referred to as a Down bending wire) 8d, which are used for up and down bending operation, and a pair of a leftward-acting bending wire (hereinafter referred to as a Left bending wire) 8l and a rightward-acting bending wire (hereinafter referred to as a Right bending wire) 8r, which are used for left and right bending operation.

According to the present embodiment, the longitudinal axis of the pulley 11 and longitudinal axis of the motor 12 intersect each other. Specifically, a drive shaft of the motor 12 is placed at a preset location in the grasping portion 3a, being in such a positional relationship as to be parallel to the longitudinal axis of the grasping portion 3a. A motor shaft 12b of the motor 12 and a pulley shaft 11b, which is a rotating shaft of the pulley 11, are set to such a positional relationship as to be orthogonal to each other. Also, the pulley 11 and the motor 12 are placed, on both sides of the partition plate 59, in different spaces in the operation section 3 partitioned by the partition plate 59.

The driving force of the motor 12 is configured to be transmitted to the pulley 11 via a driving force transmission mechanism section 15. The driving force transmission mechanism section 15 includes a first bevel gear 16 and a second bevel gear 17.

The first bevel gear 16 is integrally fixed to an axial portion 12a of the motor 12 while the second bevel gear 17 is integrally fixed to an axial portion 11a of the pulley 11. With this configuration, the driving force of the motor 12 is transmitted to the axial portion 11 a via the bevel gears 16 and 17, causing the pulley 11 to rotate axially.

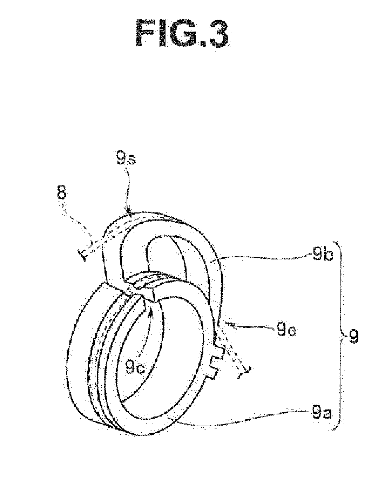

The rotating bodies 9 are elastically deformable and are each equipped with an annular portion 9a and a rotation amount adjustment portion 9b, for example, as shown in Fig. 3. A gap 9c is formed in the annular portion 9a of the rotating body 9. A wire guide portion (not shown) is formed in the annular portion 9a and the rotation amount adjustment portion 9b. The wire guide portion is configured into a preset shape so as to guide the wire 8 smoothly from a winding start position 9s to a winding end position 9e. The four rotating bodies 9u, 9d, 91, and 9r are placed with a preset loose fit around an outer circumferential face of the pulley 11 and configured to enter a rotating state independently of one another.

The suspension frame 13 is placed in a free space on the distal end side of the operation section body 3b, maintaining a preset positional relationship.

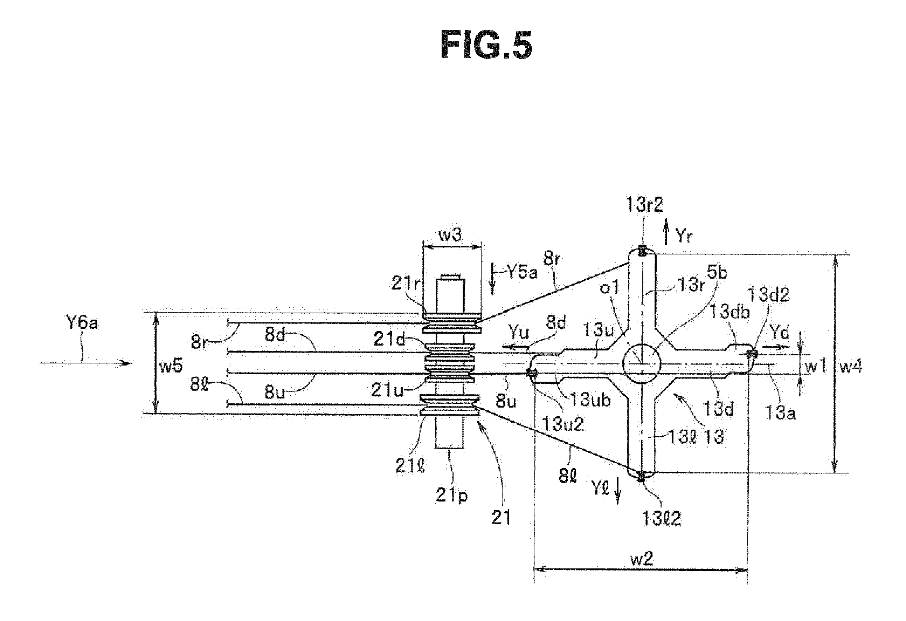

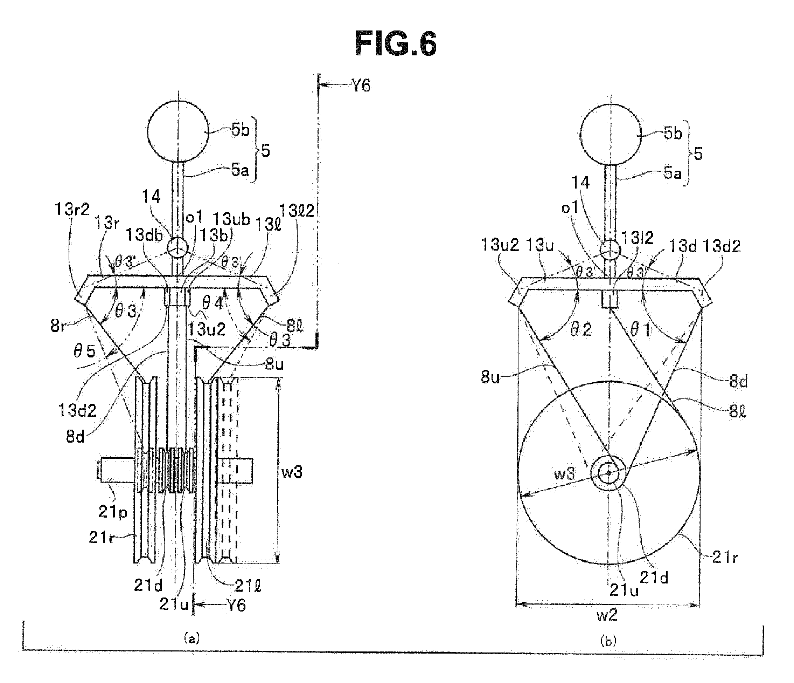

As shown in Fig. 5 as well as in a plan view of Fig. 6(a) and side view of Fig. 6(b), the suspension frame 13 is made up of four frames 13u, 13d, 131, and 13r equal in length from a center o1 to an end portion and configured to be substantially cross-shaped. An upward-acting frame (hereinafter referred to as an Up frame) 13u and a downward-acting frame (hereinafter referred to as a Down frame) 13d, corresponding to a pair of bending wires 8u and 8d, respectively, are placed on a straight line on opposite sides of the axial portion 5a. An Up wire mounting portion 13u2 is provided in the end portion of the Up frame 13u and a Down wire mounting portion 13d2 is provided in the end portion of the Down frame 13d.

On the other hand, a leftward-acting frame (hereinafter referred to as an Left frame) 131 and a rightward-acting frame (hereinafter referred to as a Right frame) 13r, corresponding to a pair of bending wires 8l and 8r, respectively, are placed orthogonally to a center line of upward-acting and downward-acting frames (hereinafter referred to as a frame center line) 13a on a straight line on opposite sides of the axial portion 5a. A Left wire mounting portion 1312 is provided in the end portion of the Left frame 131 and a Right wire mounting portion 13r2 is provided in the end portion of the Right frame 13r.

Incidentally, the plan view in Fig. 6 (a) shows the suspension frame 13 and the guide rollers 21 as viewed in the direction of the arrow Y6a in Fig. 5 while the side view in Fig. 6 (b) is a diagram as viewed in the direction of the arrow Y6- Y6 in Fig. 5 (a) . Reference character 5b denotes a finger pad spherical in shape. The finger pad 5b is integrally fixed to a tip of the axial portion 5a.

The Up frame 13u is equipped in the end portion with an Up frame tip flexing portion 13ub folded in one direction with respect to the frame center line 13a while the Down frame 13d is equipped in the end portion with a Down frame tip flexing portion 13db folded in another direction with respect to the frame center line 13a.

The Up wire mounting portion 13u2 is provided in the Up frame tip flexing portion 13ub and the Down wire mounting portion 13d2 is provided in the Down frame tip flexing portion 13db. Consequently, spacing wl between the Up wire mounting portion 13u2 and the Down wire mounting portion 13d2 in a direction orthogonal to the longitudinal axis of the operation section 3 is set to a preset size.

Incidentally, the Up frame 13u, the Up wire mounting portion 13u2, and the like are set by taking into consideration the tilting direction of the operation lever 5 and the bending direction of the bending portion 2b. The present embodiment is configured such that when the operation lever 5 is tilted in the direction of the arrow Yu in Fig. 1, the Up wire mounting portion 13u2 swings and tilts in a direction of an arrow Yu in Fig. 5, causing the bending portion 2b to bend upward. Similarly, when the operation lever 5 is tilted in the direction of the arrow Yd in Fig. 1, the Down wire mounting portion 13d2 swings and tilts in a direction of an arrow Yd in Fig. 5, causing the bending portion 2b to bend downward. Also, when the operation lever 5 is tilted in the direction of the arrow Y1 in Fig. 1, the Left wire mounting portion 1312 swings and tilts in a direction of an arrow Y1 in Fig. 5, causing the bending portion 2b to bend leftward. Also, when the operation lever 5 is tilted in the direction of the arrow Yr in Fig. 1, the Right wire mounting portion 13r2 swings and tilts in a direction of an arrow Yr in Fig. 5, causing the bending portion 2b to bend rightward.

According to the present embodiment, the suspension frame 13 is placed at a preset location in the operation section 3 such that the frame center line 13a and the longitudinal axis of the grasping portion 3a will be parallel to each other.

As shown in Figs. 2 and 5, the guide roller set 21 includes a roller shaft 21p and four guide rollers 21u, 21d, 211, and 21r. The roller shaft 21p is a support body and is, for example, cylindrical. The four guide rollers 21u, 21d, 211, and 21r are rotatably placed on the roller shaft 21p.

The four guide rollers 21u, 21d, 211, and 21r correspond, respectively, to the four bending wires 8u, 8d, 81, and 8r. The four guide rollers 21u, 21d, 211, and 21r are spaced away from the pulley 11 and the suspension frame 13 by a preset distance. The four guide rollers 21u, 21d, 211, and 21r are mounting path setting members which lead the four bending wires 8u, 8d, 81, and 8r to the wire mounting portions 13u2, 13d2, 1312, and 13r2 of the suspension frame 13.

The roller shaft 21p is placed at a preset location directly under the axial portion 5a in such a positional relationship as to be orthogonal to the longitudinal axis of the grasping portion 3a. A center of the roller shaft 21p is located on a central axis of the axial portion 5a which is in an upright state.

The bending wires 8u, 8d, 81, and 8r are configured to have their running paths changed by the respective guide rollers 21u, 21d, 211, and 21r and subsequently reach the Up wire mounting portion 13u2, the Down wire mounting portion 13d2, the Left wire mounting portion 1312, and the Right wire mounting portion 13r2 of the suspension frame 13, respectively.

The guide rollers 21 will be described with reference to Fig. 5.

Incidentally, to illustrate positional relationship between the individual bending wires 8u, 8d, 81, and 8r and the respective wire mounting portions 13u2, 13d2, 1312, and 13r2, the suspension frame 13 is shown as being displaced rightward in Fig. 5 from the roller shaft 21p.

As shown in Fig. 5, the four guide rollers 21u, 21 d, 211, and 21r are arranged on the roller shaft 21p in the order--the guide rollers 21r, 21d, 21u, and 211--as indicated by an arrow Y5a in Fig. 5.

The guide rollers 21r and 211 placed on both ends of the roller shaft 21p differ in diameter size or width size from the guide rollers 21d and 21u placed on both sides of the center of the roller shaft 21p and inner sides of the guide rollers 21r and 211.

According to the present embodiment, the Left guide roller 211 and the Right guide roller 21r are identical in diameter size and width size while the Up guide roller 21u and the Down guide roller 21d are identical in diameter size and width size. The guide rollers 211 and 21r are set to be larger by preset amounts in the diameter size and width size than the guide rollers 21 u and 21 d.

According to the present embodiment, with each of the frames 13u, 13d, 131, and 13r of the suspension frame 13 placed horizontally, the following relationships are established among wire angles of the individual bending wires 8u, 8d, 81, and 8r which extend from the respective wire mounting portions 13u2, 13d2, 1312, and 13r2 and enter the respective guide rollers 21u, 21d, 211, and 21r.

Let $\theta 1$ denote a wire angle at which the Down bending wire 8d enters the Down guide roller 21d, let $\theta 2$ denote a wire angle at which the Up bending wire 8u enters the Up guide roller 21u, and let $\theta 3$ denote a wire angle at which the Left bending wire 81 enters the Left guide roller 211 and an angle at which the Right bending wire 8r enters the Right guide roller 21r.

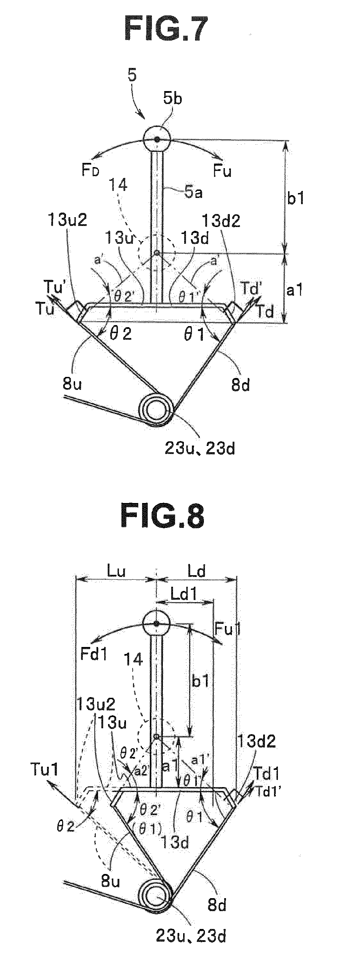

Also, an angle $\theta 1'$ is a tilt angle of a straight line joining a center of a universal joint 14 and the downward-acting wire mounting portion 13d2 as shown in Fig. 7. An angle $\theta 2'$ is a tilt angle of a straight line joining the center of the universal joint 14 and the upward- acting wire mounting portion 13u2 as shown in Fig. 7. As shown in Fig. 6, an angle $\theta 3'$ is a tilt angle of a chain double- dashed line joining the center of the universal joint 14 and a preset point of the Up frame 13u, a tilt angle of a chain double- dashed line joining the center of the universal joint 14 and a preset point of the Down frame 13d, a tilt angle of a chain double- dashed line joining the center of the universal joint 14 and a preset point of the Left frame 131, and a tilt angle of a chain double- dashed line joining the center of the universal joint 14 and a preset point of the Right frame 13r. The following relationship is established among the tilt angles.

$$\theta1' + \theta1 > \theta2' + \theta2 > \theta3' + \theta3$$

With the angles θ1, θ2, and θ3 set as described above, for example, an amount of upward operation force and an amount of downward operation force are as shown below. That is, the following balance equation is derived from the diagram shown in Fig. 7.

$$Fu \cdot b1 = Tu \cdot \sin(\theta2' + \theta2) \cdot a' \ ... \ (1)$$

$$Fd \cdot b1 = Td \cdot \sin(\theta1' + \theta1) \cdot a' \ ... \ (2)$$

where Fu: amount of upward tilting operation force
Fd: amount of downward tilting operation force
a': distance from the Up wire mounting portion and the Down wire mounting portion to the center of the universal joint
b1: distance from the center of the universal joint in the axial portion to a center of the finger pad
Tu: amount of upward wire pulling force
Td: amount of downward wire pulling force
From equation (1), Fu can be expressed as follows.

$$Fu = Tu \cdot \sin(\theta2' + \theta2) \cdot a' \ / \ b1 \ ... \ (3)$$

Also, from equation (2), Fd can be expressed as follows.

$$Fd = Td \cdot \sin(\theta1' + \theta1) \cdot a' \ / \ b1 \ ... \ (4)$$

On the pulling member operation apparatus 10, Tu and Td are equal (Tu = Td).
Therefore, equations (3) and (4) can be expressed as follows.

$$Fu = D \cdot \sin(\theta2' + \theta2) \ (where \ D = Tu \cdot a' \ / \ b1) \ ... \ (5)$$

$$Fd = D \cdot \sin(\theta1' + \theta1) \ (where \ D = Td \cdot a' \ / \ b1) \ ... \ (6)$$

Also, the relationship described above exists between the angle (θ1' + θ1) and the angle (θ2' + θ2) . Thus, the following relationship holds between Fu and Fd.

$$Fu > Fd$$

That is, the smaller the angle θ, the larger the amounts of tilting operation force. According to the present embodiment, since the relationship described above has been established among the angle (θ1' + θ1), the angle (θ2' + θ2), and the angle (θ3' + θ3), a relationship among the amount of upward tilting operation force Fu, the amount of downward tilting operation force Fd, the amount of leftward tilting operation force Fl, and the amount of rightward tilting operation force Fr is as follows.

$$Fl = Fr > Fu > Fd$$

In this way, according to the present embodiment, the amount of operation force required to bend the bending portion 2b rightward by operating and tilting the operation lever 5 rightward or the amount of operation force required to bend the bending portion 2b leftward by operating and tilting the operation lever 5 leftward is set to be the largest.

If a maximum outside diameter of guide rollers 211 and 21r is w3, the relationship of w2 > w3 is established between the maximum outside diameter w3 and the spacing w2 between the upward-acting wire mounting portion 13u2 and downward-acting wire mounting portion 13d2 in a direction of the longitudinal axis of the operation section 3.

Also, spacing between the guide roller 21u and the guide roller 21d is set to w1 which is the spacing between the upward- acting wire mounting portion 13u2 and the downward- acting wire mounting portion 13d2. Furthermore, the relationship w4 > w5 is established, where w4 is spacing between the leftward- acting wire mounting portion 1312 and the rightward- acting wire mounting portion 13r2 while w5 is spacing between an outer end of the leftward- acting guide roller 211 and outer end of the rightward- acting guide roller 21r placed around the roller shaft 21p.

Incidentally, the rotating bodies 9r, 9d, 9u, and 91 are placed on the pulley 11 in this order as indicated by an arrow Y4a in Fig. 4.

Now, the respective running paths of the bending wires 8u, 8d, 8l, and 8r in the operation section 3 will be described with reference to Figs. 2, 4, and 5.

As shown in Fig. 5, the respective proximal end portions of the four bending wires 8u, 8d, 81, and 8r are fixed to the wire mounting portions 13u2, 13d2, 1312, and 13r2 which exist at preset locations of the suspension frame 13.

On the other hand, respective distal end portions of the individual bending wires 8u, 8d, 81, and 8r are fixed to locations corresponding to up, down, left, and right positions of distal bending pieces (not shown) of the bending portion 2b. The distal bending pieces are bending pieces which make up the most distal part of a bending portion set configured to bend in the up, down, left, and right directions by linking multiple bending pieces (not shown) of the bending portion 2b.

In the insertion portion 2, the individual bending wires 8u, 8d, 81, and 8r are passed advanceably/retractably into respective guides 24 of coiled pipes made, for example, of metal and provided with through holes.

As shown in Figs. 2, 4, and 5, the bending wires 8u, 8d, 81, and 8r fixed to the distal bending pieces are extended into the operation section 3 via the respective guides 24.

The individual bending wires 8u, 8d, 81, and 8r are wound, respectively, around the rotating bodies 9u, 9d, 91, and 9r placed on the pulley 11. That is, the individual bending wires 8u, 8d, 81, and 8r are wound around the respective rotating bodies 9u, 9d, 91, and 9r starting from respective winding start positions 9s of the rotating bodies 9u, 9d, 91, and 9r so as to reach a preset slackened state. Subsequently, the individual bending wires 8u, 8d, 81, and 8r are led out from winding end positions 9e of the respective rotating bodies 9u, 9d, 91, and 9r toward the respective guide rollers 21u, 21d, 211, and 21r.

The individual bending wires 8u, 8d, 81, 8r led out of the respective rotating bodies 9u, 9d, 91, and 9r are led to the respective guide rollers 21u, 21d, 211, and 21r. After having their wire running paths changed, the bending wires 8u, 8d, 81, 8r are led to the wire mounting portions 13u2, 13d2, 1312, and 13r2 installed in the suspension frame 13. Then, the respective proximal end portions of the individual bending wires 8u, 8d, 81, and 8r are fixed to the wire mounting portions 13u2, 13d2, 1312, and 13r2.

As described above, the guide rollers 211 and 21r are set to be larger in width size than the guide rollers 21u and 21d and the spacing w4 is set to be larger than the spacing w5. Consequently, the bending wires 8l and 8r are led to the wire mounting portions 1312 and 13r2 by passing through the guide rollers 211 and 21r smoothly.

Incidentally, the axial portion 5a of the operation lever 5 and a convex frame portion 13f, which corresponds to a central axis of the suspension frame 13, are mounted and fixed coaxially via the universal joint 14 pivotably disposed on a frame (not shown) . When the axial portion 5a of the operation lever 5 is in an upright state as shown in Fig. 6, all the bending wires 8u, 8d, 81, and 8r extending from the respective guide rollers 21u, 2 1 d, 211, and 21r and heading toward the suspension frame 13 are in a predetermined slackened state.

Now, operation of the endoscope 1 will be described.

The operation involved when the operator bends the bending portion 2b upward, for example, will be described.

By gripping the grasping portion 3 a with the left hand and placing the ball of his/her thumb on the finger pad 5b of the operation lever 5, the operator operates and tilts the axial portion 5 a in the direction of the arrow Yu in Fig. 1. As a result of the tilting operation of the operation lever 5, the suspension frame 13 tilts, causing the Up bending wire 8u fixed to the Up wire mounting portion 13u2 to change gradually from a slackened state to a pulled state. On the other hand, the other bending wires 8d, 81, and 8r change to a further slackened state.

Therefore, out of the individual bending wires 8u, 8d, 81, and 8r which have been wound, in a slackened state, around the respective rotating bodies 9u, 9d, 91, and 9r on the pulley 11, only the Up bending wire 8u is pulled. Consequently, the gap 9c of a rotating body 9u for the upward- acting bending wire (hereinafter referred to as an Up rotating body) is narrowed against an elastic force and reduced in diameter, bringing the Up rotating body 9u and the pulley 11 into close contact with each other. As a result, frictional resistance is generated between the Up rotating body 9u and the pulley 11, causing the Up rotating body 9u to rotate in a same direction as the pulley 11 by slipping over the pulley 11. Consequently, the Up bending wire 8u placed closer to the insertion portion 2 than the Up rotating body 9u is pulled and moved along with the rotation of the Up rotating body 9u, causing the bending portion 2b to start the act of bending upward.

Now, if the operator keeps operating and tilting the axial portion 5a in the same direction continuously so as to bring the Up rotating body 9u into closer contact with the pulley 11, the Up rotating body 9u in close contact further comes into closer contact with the pulley 11, increasing frictional force. Consequently, the Up bending wire 8u placed closer to the insertion portion 2 than the Up rotating body 9u is pulled and moved further, causing the bending portion 2b to bend further upward.

On the other hand, if the operator maintains tilt position of the operation lever 5, an adhesive force between the Up rotating body 9u and the pulley 11 is maintained. Then, the movement stops, with a pulling force produced on the Up bending wire 8u placed on the distal end side of the Up rotating body 9u.

At this time, the bending wires 8d, 8l, and 8r are in a slackened state. Therefore, as the operation lever 5 is kept in the tilted operating state, the bending portion 2b is kept in a bent state corresponding to the tilting operation, with the Up bending wire 8u kept in a pulled state and the bending wires 8d, 8l, and 8r kept in the slackened state.

According to the present embodiment, the wire angles at which the bending wires 8u, 8d, 8l, and 8r fixed to the wire mounting portions 13u2, 13d2, 1312, and 13r2 of the suspension frame 13 enter the guide rollers 21u, 21d, 211, and 21r are set separately to preset relationships as described above.

Therefore, if the operator operates and tilts the operation lever 5, for example, rightward by mistake while intending to bend the bending portion 2b upward, the operator can sense a difference in the amount of operation force on the operation lever 5 and thereby recognize that the operation lever 5 has been operated in a direction different from the upward direction. In the embodiment described above, the relationship of angle $\theta 1 >$ angle $\theta 2 >$ angle $\theta 3$ is established among the wire angles $\theta 1$, $\theta 2$, and $\theta 3$ to obtain the relationship of Fl = Fr > Fu > Fd.

However, the relationship Fl = Fr > Fd > Fu may be obtained by establishing the relationship of angle $\theta 2 >$ angle $\theta 1 >$ angle $\theta 3$.

As an example, the bending wires 8 are wound around the guide rollers 21 in a direction opposite the direction described above, for example, as in the case of the bending wires 8u and 8d indicated by broken lines in Fig. 6(b). This provides the relationship of angle $\theta 2 >$ angle $\theta 1 >$ angle $\theta 3$ and thereby provides the relationship Fl = Fr > Fd > Fu.

However, this configuration involves placing other guide rollers further on the proximal end side of the operation section 3 than the guide rollers 21 to change the wire running paths.

Also, out of the rightward- acting guide roller 21r and the leftward- acting guide roller 211 placed on the guide shaft 21p, for example, placement location of the leftward- acting guide roller 211 may be offset sideways as indicated by broken lines in Fig. 6 (a), setting an angle $\theta 4$ larger than the angle $\theta 3$ to provide the relationship of angle $\theta 1 >$ angle $\theta 2 >$ angle $\theta 4 >$ angle $\theta 3$ and thereby obtain the relationship Fd > Fu > Fl > Fr.

Also, the angle $\theta 3$ may be set to an angle $\theta 5$ larger than the angle $\theta 1$ by setting the rightward- acting guide roller 21r and the first leftward- acting guide roller 211 equal in diameter size to the guide rollers 21u and 21d as indicated by chain double- dashed lines in Fig. 6 (a) . This provides the relationship of angle $\theta 5 >$ angle $\theta 1 >$ angle $\theta 2$, and thus the relationship Fu > Fd > Fl = Fr.

In this way, the amount of operation force of the operation lever 5 in each tilting direction is changed by setting the wire angles of the bending wires 8u, 8d, 8l, and 8r to preset angles, where the bending wires 8u, 8d, 8l, and 8r enter the guide rollers 21u, 21d, 211, and 21r by being mounted on the wire mounting portions 13u2, 13d2, 1312, and 13r2.

Consequently, when operating and tilting the operation lever 5, the operator can understand the tilting direction by recognizing a difference in the feel of the operation lever 5. This makes it possible to improve ease of bending operation.

Incidentally, in the embodiment described above, there is a difference between the amount of upward operation force Fu and the amount of downward operation force Fd. This might cause the operator to feel something odd. In such a case, the following relationship may be established by adjusting the length of the frame 13u of the suspension frame 13, as shown in Fig. 8, such that Fu = Fd.

$$Fl = Fr > Fu = Fd$$

With reference to Fig. 8, description will be given below of how to obtain the above relationship by bringing the amount of upward operation force Fu into coincidence with the amount of downward operation force Fd.

As described above, balance between the amount of upward operation force and the amount of Up bending wire pulling force as well as balance between the amount of downward operation force and the amount of Down bending wire pulling force are achieved as follows.

$$Fu1 \cdot b1 = Tu1 \cdot \sin(\theta2' + \theta2) \cdot a1' \ ... \ (11)$$

$$Fd1 \cdot b1 = Td1 \cdot \sin(\theta1' + \theta1) \cdot a2' \ ... \ (12)$$

where Fu1: amount of upward tilting operation force
Fd1: amount of downward tilting operation force
b1: distance from the center of the universal joint in the axial portion to the center of the finger pad
Tu1: amount of upward wire pulling force
Td1: amount of downward wire pulling force
a1': distance from the Down wire mounting portion to the center of the universal joint
a2': distance from the Up wire mounting portion to the center of the universal joint
From Equation (11), Fu1 can be expressed as follows.

$$Fu1 = Tu1 \cdot \sin(\theta2' + \theta2) \cdot a1' / b1 \ ... \ (13)$$

Also, from Equation (12), Fd1 can be expressed as follows.

$$Fd1 = Td1 \cdot \sin(\theta1' + \theta1) \cdot a2' / b1 \ ... \ (14)$$

On the pulling member operation apparatus 10, Tu1 and Td1 are equal (Tu1 = Td1). Therefore, equations (13) and (14) can be expressed as follows.

$$Fu1 = D \cdot \sin(\theta2' + \theta2) \ (\text{where } D = Tu1 \cdot a1' / b1) \ ... \ (5)$$

$$Fd1 = D \cdot \sin(\theta1' + \theta1) \ (\text{where } D = Td1 \cdot a1' / b1) \ ... \ (6)$$

Now, to obtain the above relationship, i.e., to bring the amount of upward operation force Fu to match the amount of downward operation force Fd, the relationship sin (θ2' + θ2) = sin (θ1' + θ1) can be satisfied. That is, the wire angle (θ2' + θ2) is changed to the angle (θ1'+ θ1) . Therefore, the length of the frame 13u is adjusted and set, as indicated by a solid line, so as to change the wire angle (θ2' + θ2) to the angle (θ1'+ θ1) .
Consequently, the amount of upward tilting operation force of the operation lever 5 can be made equal to the amount of downward tilting operation force to obtain better operability.
Incidentally, an example of adjusting the amount of upward tilting operation force by reducing the length of the Up frame 13u has been shown in the embodiment described above. However, the frame to be adjusted is not limited to the Up frame 13u, and the amount of upward tilting operation force may be adjusted by adjusting lengths of the Down frame 13d, the Left frame 13l, and the Right frame 13r.
Also, according to the embodiment described above, the medical apparatus equipped with a bending portion is an endoscope. However, the medical apparatus equipped with a bending portion is not limited to an endoscope, and may be a sliding tube used to introduce an endoscope into the body, a treatment instrument inserted into a treatment instrument channel of an endoscope, or the like.
Note that the present invention is not limited to the embodiment described above and may be modified in various forms without departing from the spirit or scope of the invention.
The present application is filed claiming the priority of Japanese Patent Application No. 2011-042552 filed in Japan on February 28, 2011, and the above described disclosure is incorporated by reference in the present description, claims and drawings.

**Claims**

1.  A medical apparatus equipped with a bending portion as a medical apparatus endoscope, comprising:

    a running path changing member adapted to change running paths of a plurality of pulling members extended from bending pieces constituting the bending portion and led into an operation section;
    a suspension frame made up of a plurality of frames and configured such that a proximal end portion of a pulling member whose running path is changed by the running path changing member is fixedly mounted on a pulling member mounting portion provided at an end portion of each frame; and
    an operation lever integrally fixed to the suspension frame and provided with an axial portion which is installed by protruding orthogonally to a longitudinal axis of an operation section body constituting the operation section and is adapted to undergo a tilting operation,
    wherein an angle at which the pulling member fixedly mounted on the pulling member mounting portion enters the running path changing member is set for each of the pulling members.

2.  The medical apparatus equipped with a bending portion according to claim 1, wherein the running path changing member and the frames of the suspension frame serve as an angle adjustment portion for setting the angle of entering the running path changing member.

3.  The medical apparatus equipped with a bending portion according to claim 1 or 2, wherein the plurality of pulling members are an upward pulling member, a downward pulling member, a leftward pulling member and a rightward pulling member, and
    an amount of upward operation force, an amount of downward operation force, an amount of leftward operation force and an amount of rightward operation force when the operation lever is operated to tilt are changed by setting diameter sizes of the running path changing member which correspond to the plurality of pulling members.

4.  The medical apparatus equipped with a bending portion according to claim 1 or 2, wherein an amount of upward operation force, an amount of downward operation force, an amount of leftward operation force and an amount of rightward operation force when the operation lever is operated to tilt are changed by adjusting lengths of the frames of the suspension frame which correspond to the plurality of pulling members.

5.  The medical apparatus equipped with a bending portion according to claim 1 or 2, wherein the amount of upward operation force is smaller than the amount of downward operation force.

6.  An endoscope as a medical apparatus endoscope equipped with a bending portion, comprising:

    a running path changing member adapted to change running paths of a plurality of pulling members extended from bending pieces constituting a bending portion and led into an operation section;
    a suspension frame made up of a plurality of frames and configured such that a proximal end portion of a pulling member whose running path is changed by the running path changing member is fixedly mounted on a pulling member mounting portion provided at an end portion of each frame; and
    an operation lever integrally fixed to the suspension frame and provided with an axial portion which is installed by protruding orthogonally to a longitudinal axis of an operation section body constituting the operation section and is adapted to undergo a tilting operation,
    wherein an angle at which the pulling member fixedly mounted on the pulling member mounting portion enters the running path changing member is set for each of the pulling members.

# FIG.1

**FIG.2**

1

2

4

5b
5a
5
7
14
13f
13
21
11
9
10
32
31
Y4
8 12
24
5
21p
21ℓ
3a
3b
3
12a

# FIG.3

# FIG.4

EP 2 649 921 A1

FIG.5

## FIG.6

# FIG.7

# FIG.8

<table>
<tr><td colspan="2" align="center">**INTERNATIONAL SEARCH REPORT**</td><td colspan="2">International application No.<br><br>PCT/JP2012/053244</td></tr>
</table>

A.   CLASSIFICATION OF SUBJECT MATTER
*A61B1/00*(2006.01)i, *G02B23/24*(2006.01)i


According to International Patent Classification (IPC) or to both national classification and IPC

B.   FIELDS SEARCHED

Minimum documentation searched (classification system followed by classification symbols)
A61B1/00, G02B23/24



Documentation searched other than minimum documentation to the extent that such documents are included in the fields searched
Jitsuyo Shinan Koho          1922-1996   Jitsuyo Shinan Toroku Koho   1996-2012
Kokai Jitsuyo Shinan Koho    1971-2012   Toroku Jitsuyo Shinan Koho   1994-2012

Electronic data base consulted during the international search (name of data base and, where practicable, search terms used)



C.   DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2003-325437 A  (Olympus Optical Co., Ltd.),<br>18 November 2003 (18.11.2003),<br>entire text; fig. 1 to 10<br>& US 2003/0092965 A1 | 1-2,6<br>3,5<br>4 |
| Y<br>A | JP 2005-13320 A  (Olympus Corp.),<br>20 January 2005 (20.01.2005),<br>paragraphs [0060] to [0071], [0087] to [0091];<br>fig. 5 to 6, 9<br>(Family: none) | 3,5<br>1-2,4,6 |
| A | JP 2004-321697 A  (Olympus Corp.),<br>18 November 2004 (18.11.2004),<br>paragraphs [0001] to [0058]; fig. 1 to 7<br>(Family: none) | 1-6 |

☒   Further documents are listed in the continuation of Box C.        ☐   See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    26 March, 2012 (26.03.12) | Date of mailing of the international search report<br>    03 April, 2012 (03.04.12) |
|---|---|
| Name and mailing address of the ISA/<br>   Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2012/053244

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2009-101076 A  (Olympus Corp.),<br>14 May 2009 (14.05.2009),<br>entire text; fig. 1 to 7<br>(Family: none) | 1-6 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000051146 A
- JP 2004321612 A
- JP 2005279118 A
- JP 2003325437 A
- JP 2011042552 A